# DEMANDE DE BREVET EUROPEEN

(11) **EP 1 541 097 A1**
(43) Date de publication de la demande: **15.06.2005**
(21) Numéro de dépôt: 04292741.8
(22) Date de dépôt: 19.11.2004
(51) Int. Cl.: A61F 5/02

(54) **Elément de connexion multi-composants, réglable et démontable, et orthèse contenant un tel élément**

(30) Priorité: 12.12.2003 FR 0314606
(71) Demandeur: Etablissements Proteor, Société Anonyme dite:, 21850 Saint Apollinarie (FR)
(72) Inventeur: Dauny, Gérald, 21420 Savigny Lès Beaune (FR)
(74) Mandataire: Pöpping, Barbara

(57) **Abrégé**

L'invention concerne un élément de connexion multi-composants, démontable et réglable, destiné à relier deux pièces d'une orthèse, comprenant
- au moins deux tiges cylindriques (1, 2) en un matériau rigide élastique, et
- deux mâchoires (3, 4) constituées chacune d'une pièce de base (3a, 4a) comportant un moyen de fixation (5) à une pièce d'une orthèse, et au moins deux rainures (6, 7) destinées à recevoir une extrémité de chacune des tiges (1, 2), et d'un étrier (3b, 4b) destiné à être fixé, par un moyen de serrage (10), sur la pièce de base dans les rainures de laquelle sont logées les extrémités des tiges, de manière à brider les tiges en une position telle que leurs parties centrales libres, non prises entre les pièces de base et l'étrier des mâchoires, soient situées dans un même plan et de préférence sensiblement parallèles l'une à l'autre ou les unes aux autres,
ainsi qu'une orthèse contenant un tel élément.

## Description

L'invention concerne un élément de connexion multi-composants, démontable et réglable, l'utilisation d'un tel élément de connexion pour augmenter de manière réglable l'élasticité en torsion et en flexion d'une orthèse, ainsi qu'une orthèse, en particulier un corset pour la réduction tridimensionnelle dynamique d'une scoliose, contenant au moins un tel élément de connexion.

Dans le domaine des orthèses, se pose de manière générale le problème de trouver un compromis entre un soutien à la fois rigoureux et souple (élastique), d'une partie du corps humain. Des attelles ou corsets, par exemple, sont destinés à orienter de manière importante le mouvement d'une partie du corps autour d'une articulation ou à imposer à cette partie du corps une certaine contrainte en vue de la correction d'un mouvement ou d'une position pathologique.

L'immobilisation stricte ou la forte contrainte limitant le patient dans sa liberté de mouvement, sont généralement ressenties comme désagréables. Ce désagrément pourrait, dans certains cas, être atténué si l'on disposait de pièces ou de matériaux élastiques appropriés permettant une liberté de mouvement contrôlée dans certaines directions de l'espace, tout en imposant, dans d'autres directions de l'espace, des contraintes mécaniques plus fortes, indispensables à l'effet thérapeutique.

La Demanderesse a proposé dans son brevet européen EP 0 389 379 B1 un corset pour la réduction tridimensionnelle dynamique des scolioses qui, au cours de la phase d'inspiration du cycle respiratoire du patient, s'oppose à l'expansion naturelle de la cage thoracique du patient par l'avant le forçant ainsi à se voûter, ce qui amène le rachis dorsal en position de cyphose, pour normaliser ainsi la position vertébrale.

Ce corset comprend, d'une part, deux mains latérales en une matière plastique déformable élastiquement, destinées à enserrer latéralement le thorax du patient, avec une partie antérieure disposée à l'avant du thorax et une partie postérieure disposée à l'arrière de celui-ci, d'autre part, une ceinture pelvienne destinée à être rendue solidaire du bassin du patient, et au moins deux mats, ou tuteurs, latéraux en une matière semi-rigide déformable élastiquement, qui réunissent en direction verticale respectivement chacune desdites mains et la partie correspondante de la ceinture pelvienne. Ces mats, constitués d'un matériau rigide élastique, par exemple d'une résine thermoplastique renforcée par des fibres de carbone, exercent un effort de rappel en torsion sur les parties postérieures des mains lorsque celles-ci s'écartent l'une de l'autre en phase d'inspiration du thorax du patient.

Ces mats ne permettent toutefois aucun mouvement en avant ou en arrière (flexion sagittale) du corset et imposent ainsi au patient une posture peu naturelle, mal supportée en particulier par des enfants. Or, cette forte rigidité sagittale n'est pas indispensable à l'effet de Correction du corset, et un certain assouplissement du corset dans le sens avant/arrière pourrait augmenter le confort des patients et aider ceux-ci à mieux tolérer l'orthèse sans toutefois altérer l'effet thérapeutique.

Dans une telle perspective d'assouplissement élastique d'un tel corset, mais également d'autres orthèses, la Demanderesse a mis au point un élément de connexion, qui, lorsqu'il est monté en une position appropriée d'une orthèse, permet de conférer à celle-ci une certaine élasticité en torsion et en flexion selon une première direction de l'espace, tout en maintenant une rigidité importante selon une deuxième direction de l'espace, perpendiculaire à la première.

L'invention a par conséquent pour objet un élément de connexion multi-composants, destiné à relier deux pièces d'une orthèse, comprenant
- au moins deux tiges en un matériau rigide élastique, et
- deux mâchoires constituées chacune
   - d'une pièce de base comportant un moyen de fixation à une pièce d'une orthèse, et au moins deux rainures destinées à recevoir une extrémité de chacune des tiges, et
   - d'un étrier destiné à être fixé, par un moyen de serrage, sur la pièce de base dans les rainures de laquelle sont logées les extrémités des tiges, de manière à brider les tiges en une position telle que leurs parties centrales libres, non prises entre les pièces de base et les étriers des mâchoires, soient situées dans un même plan et de préférence sensiblement parallèles l'une à l'autre ou les unes aux autres.

Pour décrire plus en détail les propriétés mécaniques de l'élément de connexion de la présente invention, il est nécessaire de définir les trois mouvements élastiques de base que l'élément de connexion peut subir en réaction à une contrainte :
- Le premier de ces mouvements est la *flexion latérale,* à savoir la flexion des tiges dans le plan passant par les axes centraux de l'ensemble des tiges.
- Le deuxième mouvement est la *flexion sagittale,* à savoir la flexion des tiges selon un plan, parallèle aux tiges et perpendiculaire au plan de flexion latérale, et enfin,
- la *torsion horizontale* résultant d'une contrainte de rotation d'une des mâchoires par rapport à l'autre dans un plan perpendiculaire aux tiges, entraînant la vrille de celles-ci.

L'élément de connexion de la présente invention effectue une flexion élastique sagittale et une torsion élastique horizontale sous des contraintes relativement modérées, mais présente une résistance importante à une flexion latérale.

L'avantage de ce nouvel élément de connexion réside non seulement dans ses propriétés élastiques anisotropes, mais également dans le fait qu'il est facilement démontable et réglable par l'orthoprothésiste, par exemple grâce à un simple écartement ou rapprochement respectif des mâchoires ou grâce au remplacement et/ou à l'addition d'une ou de plusieurs tiges.

Le comportement élastique de l'élément de connexion dépendra, entre autres, des propriétés élastiques du matériau formant les tiges (module d'Young, allongement élastique), de la longueur libre des tiges, du diamètre des tiges, du nombre de tiges montées dans un même plan ou encore de l'écartement entre les tiges.

L'élasticité du matériau, la longueur libre des tiges et le diamètre des tiges ont une influence sur les trois mouvements de base décrits ci-dessus. Le système sera d'autant plus souple dans les trois directions de l'espace (flexion latérale, flexion sagittale et torsion horizontale) que le matériau est élastique, que la longueur libre des tiges est grande et que le diamètre des tiges est faible. L'orthoprothésiste pourra ainsi moduler à souhait les propriétés de l'élément en faisant varier ces paramètres et en combinant éventuellement différentes tiges. L'augmentation du nombre de tiges au-delà de 2 accroît fortement la rigidité en flexion latérale, modérément la rigidité en torsion et peu la rigidité en flexion sagittale. La modification de l'écartement entre les tiges n'influe en rien sur la flexion sagittale et sur la flexion latérale mais beaucoup sur la torsion horizontale.

Le cintrage des tiges avant ou pendant le serrage des étriers sur les pièces de base des mâchoires offre d'autres possibilités de réglage des dimensions et des propriétés mécaniques de l'élément de connexion de la présente invention. Les tiges peuvent par exemple être cintrées, dans le plan de flexion sagittale, au-delà de la déformation élastique du matériau, ou encore être cintrées, dans le plan de flexion latérale, dans ou hors déformation élastique avant le serrage des étriers. La tige convexe prend alors une longueur libre plus grande que la tige concave. Enfin, une torsion horizontale peut être imposée aux tiges avant serrage des étriers.

Le matériau formant les tiges cylindriques élastiques doit être à la fois suffisamment rigide et élastique pour remplir sa fonction de jonction souple dans un système de maintien forcé d'une partie du corps humain.

On peut citer à titre d'exemples de matériaux appropriés les alliages métalliques, par exemple l'acier à ressort ou les alliages superélastiques, ou encore les composites de résines thermoplastiques ou thermodurcissables et de fibres de carbone, ces dernières étant de préférence tressées ou tissées.

La Demanderesse a constaté que l'élément de connexion remplit particulièrement bien sa fonction de jonction souple lorsque le matériau élastique formant les tiges présente un allongement élastique compris entre 1 et 15 %, de préférence entre 2 et 8 %, ce qui est généralement le cas des alliages superélastiques mentionnés ci-dessus.

Les extrémités des tiges cylindriques en un matériau rigide élastique doivent être parfaitement fixées entre la pièce de base et l'étrier de chaque mâchoire. Le moyen de serrage permettant de fixer fermement l'étrier sur la pièce de base est de préférence une vis dans un orifice fileté prévu dans l'étrier et en une position correspondante dans la pièce de base.

Comme indiqué ci-dessus, les extrémités des tiges cylindriques viennent se loger dans des rainures prévues à cet effet dans chacune des pièces de base des mâchoires. Ces rainures ont de préférence une profondeur inférieure au diamètre des tiges de manière à ce que seule une partie de l'épaisseur des tiges disparaît dans lesdites rainures et qu'une autre partie de l'épaisseur dépasse la surface plane de la pièce de base de chaque mâchoire. L'étrier comportera alors des rainures correspondant à celles de la pièce de base. De préférence, la profondeur des rainures dans la pièce de base et dans l'étrier est légèrement inférieure au ½ diamètre des tiges.

Les extrémités de tiges et les rainures dans la pièce de base et dans l'étrier peuvent être parfaitement droites, ce qui présente l'avantage de permettre à tout moment le réglage réversible de la longueur libre des tiges par simple desserrage et resserrage du moyen de serrage. Dans ce mode de réalisation, le risque d'arrachement des tiges de la mâchoire n'est toutefois pas négligeable car les extrémités des tiges peuvent glisser dans les rainures sous l'effet d'une contrainte de traction importante.

Dans un mode de réalisation préféré de l'élément de connexion de la présente invention, ce problème est résolu grâce à des extrémités coudées des tiges. L'effet « crochet » d'un coude prévu au niveau de chacune des extrémités de tiges maintiendra celles-ci fermement accrochées entre les pièces de base et les étriers des mâchoires, à condition, bien entendu, que les rainures, destinées à recevoir ces extrémités des tiges, ont une forme coudée correspondante.

Un effet « crochet » équivalent est obtenu lorsque deux tiges sont réunies au niveau de leurs extrémités, par un arrondi en U, et deux rainures, destinées à recevoir ces extrémités de tiges réunies en U, ont une forme arrondie en U correspondante. Il s'agit là par conséquent d'un autre mode de réalisation préféré de l'élément de connexion de la présente invention.

Dans encore un autre mode de réalisation préféré de la présente invention, l'élément de connexion a une configuration modulable grâce à une multitude de rainures prévue sur plusieurs faces de chacune des mâchoires. Plus précisément, dans ce mode de réalisation, la pièce de base d'au moins une des mâchoires a une forme de polyèdre, de préférence de parallélépipède rectangle, portant, sur une de ses faces, un moyen de fixation et, sur plusieurs de ses autres faces, au moins deux rainures parallèles destinées à recevoir les extrémités des tiges. L'orthoprothésiste pourra alors choisir, en fonction de l'orientation et du déport qu'il souhaite donner aux moyens de fixation, la face de la partie de base qui recevra les extrémités des tiges et contre laquelle viendra s'appliquer l'étrier. De manière particulièrement préférée, chacune des 2, 3, 4 ou 5 autres faces de la partie de base parallélépipédique de la mâchoire comporte non pas une seule mais deux paires de rainures parallèles, formant un angle droit l'une par rapport à l'autre.

Comme il a été décrit ci-dessus en détail, la structure particulière de l'élément de connexion de la présente invention constitué d'au moins deux tiges élastiques bridées entre deux mâchoires, fait que cet élément a des propriétés élastiques qui varient en fonction du sens de la contrainte appliquée. Cette propriété peut être mise à profit pour moduler de manière sélective la rigidité d'une orthèse.

L'invention a par conséquent également pour objet l'utilisation d'un élément de connexion tel que décrit ci-dessus pour augmenter de manière réglable, l'élasticité en torsion et l'élasticité en flexion selon un plan particulier d'une orthèse, tout en maintenant une rigidité dans un autre plan, perpendiculaire au premier. Pour cela, un ou plusieurs éléments de connexion selon l'invention sont montés de préférence de manière à ce que les plans de flexion sagittale des différents éléments soient sensiblement parallèles les uns aux autres et, dans le cas d'un corset, parallèles au plan sagittal du patient.

On peut citer comme exemple d'application préféré d'un élément de connexion selon l'invention, l'utilisation dans un corset pour la réduction tridimensionnelle dynamique de la scoliose décrit dans le brevet EP 0 389 379 B1 de la Demanderesse. Dans ce cas, deux éléments de connexion selon l'invention sont montés respectivement en tant que partie intégrante de chacun des mats latéraux rigides du corset et sont orientés de manière à ce que leur plan de flexion sagittal est sensiblement parallèle au plan sagittal du patient (plan de flexion avant/arrière). Autrement dit, chacun des deux mats du corset connu est remplacé par deux demi-mats fixés respectivement sur la ceinture pelvienne et sur une main latérale, et reliés l'un à l'autre par l'élément de connexion selon l'invention. Un tel montage permet une flexion élastique dans le plan sagittal du patient tout en assurant une rigidité dans le plan latéral du patient.

Enfin, un troisième et dernier objet de l'invention est une orthèse comportant au moins un élément de connexion tel que décrit ci-dessus, et en particulier un corset pour la réduction tridimensionnelle dynamique d'une scoliose, du type de celui décrit dans le brevet EP 0 389 379 B1 de la Demanderesse, comprenant
- d'une part, deux mains latérales en une matière plastique déformable élastiquement, destinées à enserrer latéralement le thorax du patient, avec une partie antérieure disposée à l'avant du thorax et une partie postérieure disposée à l'arrière de celui-ci,
- d'autre part, une ceinture pelvienne destinée à être rendue solidaire du bassin du patient, et
- au moins deux mats latéraux en une matière semi-rigide déformable élastiquement, qui réunissent en direction verticale respectivement chacune desdites mains et la partie correspondante de la ceinture pelvienne,
ce corset étant caractérisé par le fait qu'un élément de connexion selon l'invention est monté, en tant que partie intégrante de chacun des deux mats latéraux, dans la partie libre de celui-ci, et est orienté de manière à ce que les plans latéral et sagittal dudit élément de connexion soient respectivement sensiblement parallèles aux plans latéral et sagittal du patient, de manière à permettre à celui-ci une certaine liberté de torsion horizontale et de flexion sagittale.

D'autres applications envisageables de l'élément de connexion selon l'invention sont par exemple les appuis-tête et les corsets-sièges de rééducation.

L'invention est maintenant décrite en référence aux dessins annexés, non limitatifs, dans lesquels :
- la figure 1 est une vue en perspective éclatée d'un élément de connexion selon l'invention,
- les figures 2 et 3 sont des vues en perspective d'un élément de connexion à mâchoires orientables selon un mode de réalisation préféré de l'invention,
- la figure 4 est une vue en perspective des différentes pièces formant un élément de connexion selon un autre mode de réalisation préféré de l'invention,
- la figure 5 est une vue en perspective d'un élément de connexion assemblé à partir des pièces de la figure 4,
- la figure 6 est une vue en perspective d'une partie d'un élément de connexion selon un troisième mode de réalisation préféré selon l'invention,
- la figure 7 est une vue latérale d'un élément de connexion selon l'invention incorporé dans un corset en tant que partie intégrante d'un des mats latéraux, et
- la figure 8 est une vue latérale d'une variante d'un élément de connexion selon l'invention en tant que partie intégrante d'un des mats latéraux d'un corset.

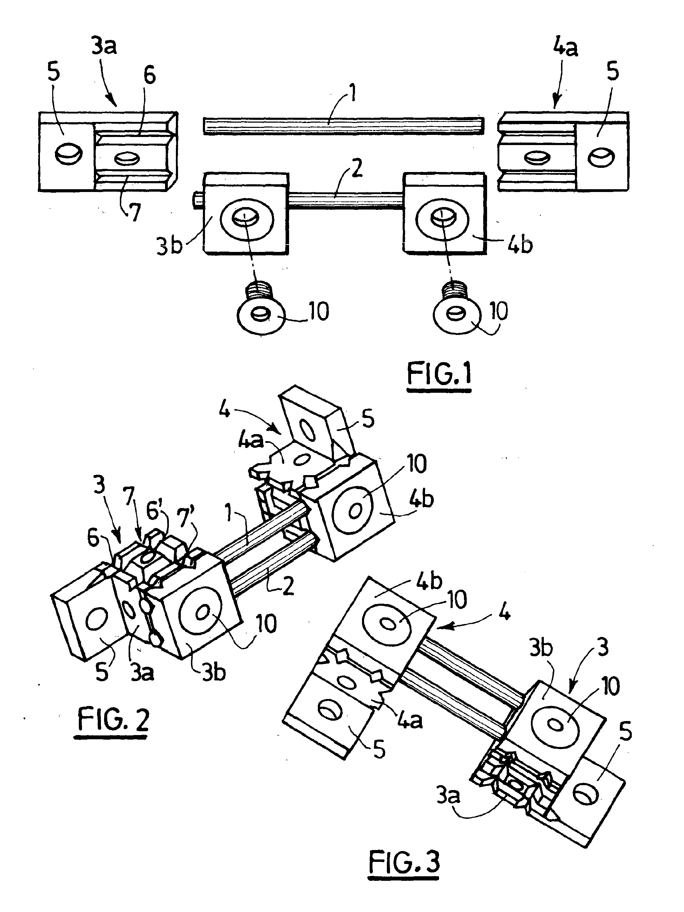

Dans la figure 1 sont représentés les différentes pièces formant, après assemblage, l'élément de connexion de la présente invention. Chaque élément de connexion comporte deux mâchoires 3, 4 constituées chacune d'une pièce de base 3a, 4a et d'un étrier 3b, 4b. Chaque pièce de base 3a, 4a est composée d'une partie de fixation 5 au corset, percée d'un orifice fileté, et d'une partie comportant deux rainures droites 6, 7 destinées à recevoir les extrémités de deux tiges 1, 2. Les deux étriers 3b, 4b aussi comportent, sur une de leurs faces, deux rainures droites 6', 7' qui se retrouveront, après fixation de l'étrier sur la pièce de base, en une position correspondante à celle des rainures 6, 7 de cette dernière, de manière à brider fermement les extrémités des deux tiges. La fixation des étriers 3b, 4b sur les pièces de base 3a, 4a se fait au moyen d'une vis 10 vissée dans des orifices 9a, 9b prévus respectivement dans l'étrier et la pièce de base.

L'élément de connexion représenté sur les figures 2 et 3 diffère de celui de la figure 1 principalement par la géométrie des deux pièces de base 3a, 4a. Celles-ci sont sensiblement cubiques et portent, sur une de leurs surfaces, une partie de fixation 5 au corset, percée d'un orifice. Quatre des cinq autres surfaces du cube portent chacune deux paires de rainures parallèles 6, 7, formant un angle droit l'une par rapport à l'autre. En considérant que chaque paire de rainures 6, 7 peut recevoir une paire de tiges 1, 2 de deux manières différentes (la partie libre des tiges dépassant d'un côté ou de l'autre de la mâchoire), il existe au total seize manières de fixer une mâchoire à deux tiges fixes, avec autant d'orientations différentes de la partie de fixation 5. Sur les figures 2 et 3, la mâchoire 3 est fixée de manière identique aux tiges 1, 2 alors que la position relative de la mâchoire 4 par rapport à la mâchoire 3 est différente.

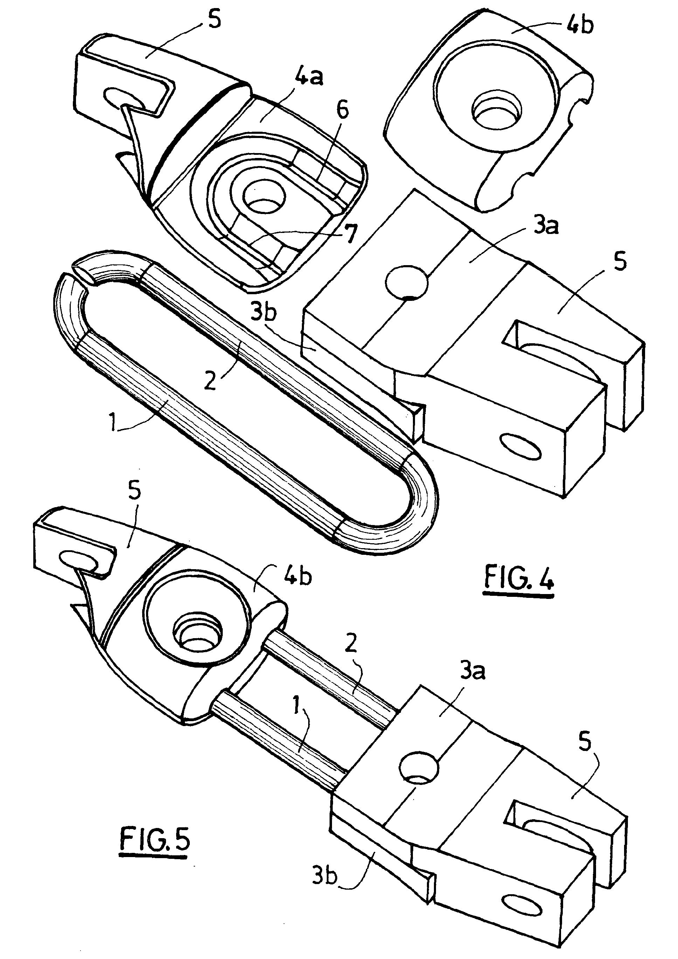

Les figures 4 et 5 illustrent un mode de réalisation de l'élément de connexion de la présente invention où deux tiges 1, 2 sont réunies au niveau de leurs extrémités par un arrondi en U. Un tel arrondi, qu'il soit fermé ou ouvert, empêche, par un effet de crochet, le glissement des tiges 1, 2 dans les rainures 6, 7 en cas de traction excessive. Les deux mâchoires sont relativement plus simples et légères que celles représentées sur les figures 2 et 3 mais imposent une orientation unique et fixe aux parties de fixation 5.

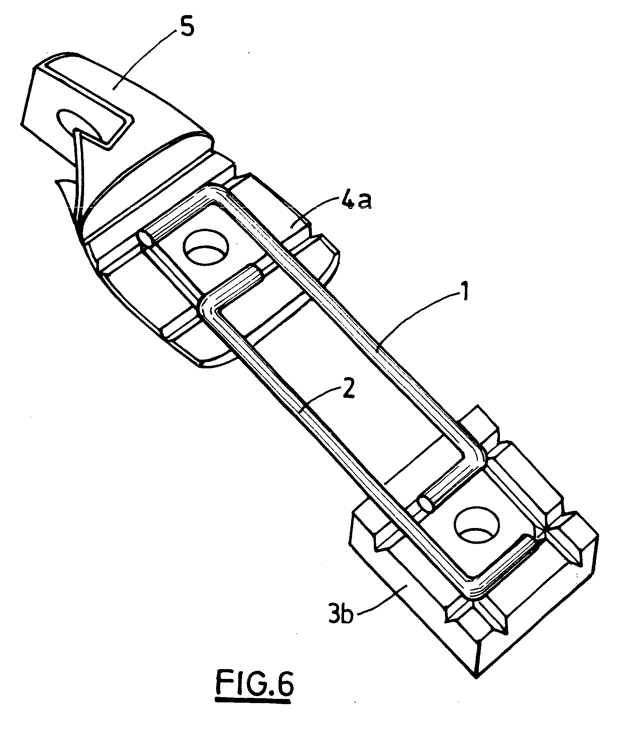

La figure 6 illustre un mode de réalisation de l'élément de connexion de la présente invention où les deux tiges 1, 2, coudées à leurs extrémités, viennent se loger dans des rainures correspondantes des mâchoires. On obtient un effet crochet analogue à celui décrit pour les figures 4 et 5.

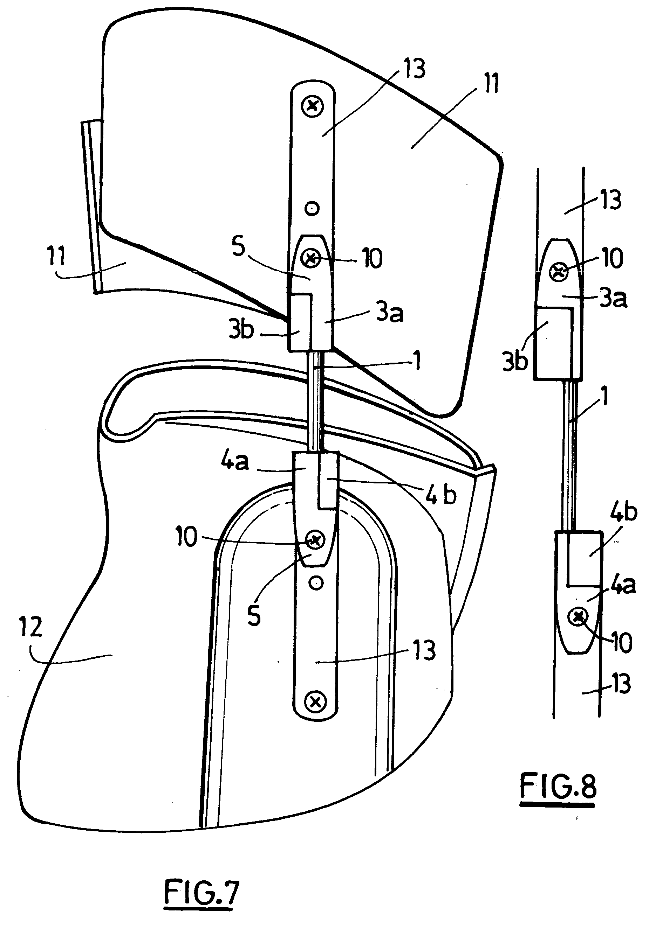

La figure 7 montre un élément de connexion monté au niveau de la partie centrale libre d'un mat latéral 13 d'un corset pour la réduction tridimensionnelle dynamique d'une scoliose, reliant la ceinture pelvienne 12 à une des mains latérales 11. L'élément de connexion est constitué de deux mâchoires, constituées chacune d'une pièce de base 3a, 4a et d'un étrier 3b, 4b, et de deux tiges 1, 2 dont une seule est visible. Dans cette position, le plan de flexion sagittale de l'élément de jonction est sensiblement parallèle au plan de symétrie sagittal du patient. La présence et la position de cet élément de connexion dans le corset augmente la souplesse de celui-ci en cas de fléchissement en avant ou en arrière du patient ou en cas de torsion latérale du thorax, pris entre les mains latérales, par rapport à la ceinture pelvienne rendue solidaire du bassin du patient. La rigidité relativement importante de l'élément de connexion dans le plan de flexion latéral, parallèle au plan de flexion latéral du patient, permet toutefois de conserver à l'ensemble une rigidité suffisante pour un soutien efficace du thorax par les deux mains d'appui.

Enfin, la figure 8 représente, de manière analogue à la figure 7, un élément de connexion selon l'invention monté au niveau de la partie centrale libre d'un mat latéral. La géométrie des mâchoires, plus précisément la faible épaisseur des pièces de base 3a, 4a et la forte épaisseur des étriers 3b, 4b, aboutit à un déport d'un des demi-mats par rapport à l'autre, et ainsi à un décalage de la main latérale (non représentée) sur laquelle est fixée le demi-mat supérieur, en avant ou en arrière par rapport à la ceinture pelvienne (non représentée) sur laquelle est fixé le demi-mat inférieur.

## Revendications

1. Elément de connexion multi-composants, démontable et réglable, destiné à relier deux pièces d'une orthèse, comprenant
• au moins deux tiges cylindriques (1, 2) en un matériau rigide élastique choisi parmi les alliages métalliques ou les composites résine/fibres de carbone, et
• deux mâchoires (3, 4) constituées chacune
- d'une pièce de base (3a, 4a) comportant un moyen de fixation (5) à une pièce d'une orthèse, et au moins deux rainures (6, 7) destinées à recevoir une extrémité de chacune des tiges (1, 2), et
- d'un étrier (3b, 4b) destiné à être fixé, par un moyen de serrage (10), sur la pièce de base (3a, 4a) dans les rainures de laquelle sont logées les extrémités des tiges (1, 2), de manière à brider les tiges en une position telle que leurs parties centrales libres, non prises entre les pièces de base (3a, 4a) et les étriers (3b, 4b) des mâchoires, soient situées dans un même plan et de préférence sensiblement parallèles l'une à l'autre ou les unes aux autres.

2. Elément de connexion selon la revendication 1, **caractérisé par le fait que** le matériau formant les tiges (1, 2) présente un allongement élastique compris entre 1 et 15 %, de préférence entre 2 et 8 %.

3. Elément de connexion selon l'une quelconque des revendications précédentes, **caractérisé par le fait que** la pièce de base (3a, 4a) d'au moins une des mâchoires a une forme de polyèdre portant, sur une de ses faces, un moyen de fixation (5) et, sur plusieurs de ses autres faces au moins deux rainures parallèles (6, 7) destinées à recevoir les extrémités des tiges (1, 2).

4. Elément de connexion selon la revendication 3, **caractérisé par le fait que** la pièce de base (3a, 4a) d'au moins une des mâchoires est un parallélépipède rectangle portant, sur une de ses faces, un moyen de fixation (5) et sur chacune des 2, 3, 4 ou 5 autres faces deux paires de rainures, les deux rainures formant chaque paire étant parallèles l'une à l'autre et les deux paires de rainures formant un angle droit l'une par rapport à l'autre.

5. Elément de connexion selon l'une quelconque des revendications précédentes, **caractérisé par le fait que** les extrémités des tiges ont une forme coudée et que les rainures, destinées à recevoir ces extrémités des tiges, ont une forme coudée correspondante.

6. Elément de connexion selon l'une quelconque des revendications 1 à 4, **caractérisé par le fait que** deux tiges sont réunies au niveau de leurs extrémités, par un arrondi en U, et que deux rainures, destinées à recevoir ces extrémités de tiges réunies en U, ont une forme arrondie en U correspondante.

7. Orthèse comportant au moins un élément de connexion selon l'une quelconque des revendications 1 à 6.

8. Orthèse selon la revendication 7, **caractérisée par le fait qu'**il s'agit d'un corset pour la réduction tridimensionnelle dynamique d'une scoliose, comprenant
- d'une part, deux mains latérales (11) en une matière plastique déformable élastiquement, destinées à enserrer latéralement le thorax du patient, avec une partie antérieure disposée à l'avant du thorax et une partie postérieure disposée à l'arrière de celui-ci,
- d'autre part, une ceinture pelvienne (12) destinée à être rendue solidaire du bassin du patient, et
- au moins deux mats latéraux (13) en une matière semi-rigide déformable élastiquement, qui réunissent en direction verticale respectivement chacune desdites mains (11) et la partie correspondante de la ceinture pelvienne (12),
un élément de connexion selon l'une quelconque des revendications 1 à 6, étant monté, en tant que partie intégrante de chacun des mats latéraux (13), dans la partie libre de celui-ci, et étant orienté de manière à ce que les plans latéral et sagittal dudit élément de connexion sont respectivement sensiblement parallèles aux plans latéral et sagittal du patient, de manière à permettre à celui-ci une certaine liberté de torsion horizontale et de flexion sagittale.
